(19) Europäisches Patentamt / European Patent Office / Office européen des brevets

(11) **EP 1 766 398 B1**

(12) **EUROPEAN PATENT SPECIFICATION**

(45) Date of publication and mention
of the grant of the patent:
**05.01.2011 Bulletin 2011/01**

(51) Int Cl.:
***G01N 33/543*** *(2006.01)*   ***C12Q 1/68*** *(2006.01)*

(21) Application number: **05752598.2**

(22) Date of filing: **17.06.2005**

(86) International application number:
**PCT/SE2005/000933**

(87) International publication number:
**WO 2006/001749 (05.01.2006 Gazette 2006/01)**

(54) **METHOD FOR DETECTING MOLECULAR SURFACE INTERACTIONS**

VERFAHREN ZUM NACHWEIS MOLEKULARER OBERFLÄCHENWECHSELWIRKUNGEN

DETECTION DES INTERACTIONS SUPERFICIELLES MOLECULAIRES

(84) Designated Contracting States:
**AT BE BG CH CY CZ DE DK EE ES FI FR GB GR HU IE IS IT LI LT LU MC NL PL PT RO SE SI SK TR**

(30) Priority: **24.06.2004 SE 0401633**
**24.06.2004 US 582743 P**

(43) Date of publication of application:
**28.03.2007 Bulletin 2007/13**

(73) Proprietor: **GE Healthcare Bio-Sciences AB**
**751 84 Uppsala (SE)**

(72) Inventors:
• **ANDERSSON, Karl**
**S-756 43 Uppsala (SE)**
• **MOBERG, Anna**
**S-757 55 Uppsala (SE)**

(74) Representative: **Stavbom, Ellen Elisabet et al**
**GE Healthcare Bio-Sciences AB**
**Patent Department**
**Björkgatan 30**
**751 84 Uppsala (SE)**

(56) References cited:
**US-A- 5 656 504**

• **SCHNEIDER B.H. ET AL: 'Highly sensitive optical chip immunoassays in human serum' BIOSENSORS & BIOELECTRONICS vol. 15, 2000, pages 13 - 22, XP002991673**
• **RICH R.L. ET AL: 'High-Resolution and High-Throughput Protocols for Measuring Drug/ Human Serum Albumin Interactions Using BIACORE' ANALYTICAL BIOCHEMISTRY vol. 296, 2001, pages 197 - 207, XP002307383**

## Description

### FIELD OF THE INVENTION

The present invention relates to the detection of molecular interactions at a solid support surface, and more particularly to a method of determining interactions between an analyte present in a sample based on a complex medium, such as a body fluid, and an immobilized binder for the analyte.

### BACKGROUND OF THE INVENTION

A variety of analytical techniques are used to characterize interactions between molecules, particularly in the context of assays directed to the detection and interaction of biomolecules. For example, antibody-antigen interactions are of fundamental importance in many fields, including biology, immunology and pharmacology. In this context, many analytical techniques involve binding of a "ligand", such as an antibody, to a solid support, followed by contacting the ligand with an "analyte", such as an antigen. Following contact of the ligand and analyte, some characteristic is measured which is indicative of the interaction, such as the ability of the ligand to bind the analyte. It is often desired that after measurement of the interaction, it should be possible to dissociate the ligand-analyte pair in order to "regenerate" free ligand, thereby enabling reuse of the ligand surface for a further analytical measurement.

Analytical sensor systems that can monitor such molecular interactions in real time are gaining increasing interest. These systems are often based on optical biosensors and usually referred to as interaction analysis sensors or biospecific interaction analysis sensors. A representative such biosensor system is the Biacore® instrumentation sold by Biacore AB (Uppsala, Sweden) which uses surface plasmon resonance (SPR) for detecting interactions between molecules in a sample and molecular structures immobilized on a sensing surface. With the Biacore® systems it is possible to determine in real time without the use of labeling not only the presence and concentration of a particular molecule in a sample, but also additional interaction parameters such as, for instance, the association rate and dissociation rate constants for the molecular interaction.

However, when the analyte is present in a complex medium, such as e.g. blood plasma, the measurements will usually be severely disturbed by species other than the analyte that are present in the plasma and interact with the solid phase surface, giving rise to non-specific binding. This leads to measurement results that are usually difficult to interpret, especially for low molecular weight analytes.

It is an object of the present invention to overcome the above-mentioned problem in detecting interactions of an analyte with a solid phase surface when the analyte is present in a complex medium.

### SUMMARY OF THE INVENTION

The above and other objects and advantages are provided by a novel method for determining interaction between an analyte with a binding agent, or ligand, immobilized to a solid support surface where the analyte is present in a complex medium containing one or more species that may also interact with the solid support surface. According to the present invention, the influence of non-specific binding at the solid support surface on the determination result may be at least substantially reduced if the monitored solid support surface is first contacted with the complex medium free from analyte directly followed by the analyte-containing complex medium. Any non-specific binding of the complex medium with the solid support will thereby at least to a substantial degree take place before the surface is contacted with the analyte-containing medium, and any additional changes that are then detected at the surface may therefore to a large extent be attributed to the binding of analyte.

In one aspect, the present invention therefore provides a method for determining interaction of an analyte with a binding agent immobilized to a solid support surface by contacting the surface with a fluid sample containing the analyte, and detecting binding events at the solid support surface, wherein the sample is based on a complex medium containing at least one species other than analyte that may interact with the solid support surface, which method is characterized in that it comprises contacting the solid support surface with sample medium free from analyte prior to contacting the surface with the analyte-containing sample.

In another aspect, the present invention provides a method for determining the plasma binding propensity of a drug, which method comprises determining the binding of a drug present (i) in a complex medium comprising blood plasma, and (ii) in a non-complex medium, to an immobilized binder for the drug, and comparing the binding of drug in the blood plasma-comprising medium with the binding of drug in the non-complex medium, wherein the determination of the binding of drug in the blood plasma-comprising medium is performed according to the first method aspect above. These and other aspects of the invention will be evident upon reference to the accompanying drawings and the following detail description

### BRIEF DESCRIPTION OF THE DRAWINGS

Figure 1 is a schematic side view of a biosensor system based on SPR.
Figure 2 is a sensorgram showing detector response versus time for the interaction between an analyte and an immobilized binder for the analyte.
Figure 3 is an overlay plot of (i), in dashed line, a sensorgram for the injection of saliva-containing buffer embedded between injections of pure buffer, and (ii), in solid line, a sensorgram for the sequential injections of pure buffer.

Figure 4 is an overlay plot of two sensorgrams, each representing three sequential injections of saliva-containing buffer.

Figure 5 is an overlay plot of (i), in solid line, a sensorgram for injection of iophenoxic acid in saliva-containing buffer embedded between injections of saliva-containing buffer, and (ii), in dashed line, a sensorgram for corresponding injections of saliva-containing buffer.

Figure 6 is an overlay plot of, in dashed lines, three corrected sensorgrams obtained by subtracting sensorgrams corresponding to sensorgram (ii) in Fig. 5 from sensorgrams corresponding to sensorgram (i) in Fig. 5, and, in solid lines, corresponding sensorgrams for injections of iophenoxic acid in pure buffer.

## DETAILED DESCRIPTION OF THE INVENTION

As mentioned above, the present invention relates to a method of reducing or eliminating the influence of non-specific binding by non-analyte components of a complex sample medium in assays or studies involving the detection of binding events at a solid support surface. Surface binding interactions may be characterized using a number of different interaction analysis techniques. Recently, label-free biosensor technology has become a powerful tool for such interaction analysis. Commercially available biosensors include the above-mentioned Biacore® system instruments which are based on surface plasmon resonance (SPR) and permit monitoring of surface interactions in real time.

The phenomenon of SPR is well known, suffice it to say that SPR arises when light is reflected under certain conditions at the interface between two media of different refractive indices, and the interface is coated by a metal film, typically silver or gold. In the Biacore® instruments, the media are the sample and the glass of a sensor chip which is contacted with the sample by a microfluidic flow system. The metal film is a thin layer of gold on the chip surface. SPR causes a reduction in the intensity of the reflected light at a specific angle of reflection. This angle of minimum reflected light intensity varies with the refractive index close to the surface on the side opposite from the reflected light, in the Biacore® system the sample side.

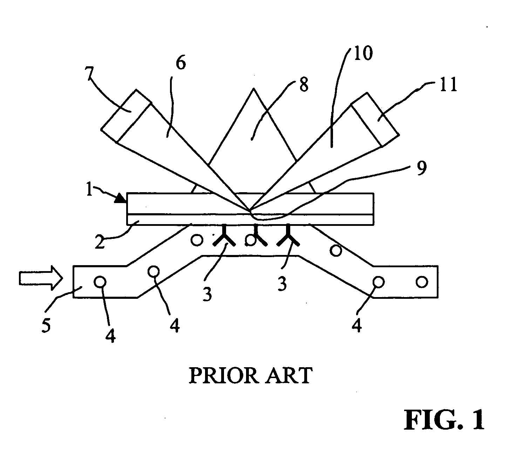

A schematic illustration of the Biacore® system is shown in Fig. 1. Sensor chip 1 has a gold film 2 supporting capturing molecules (ligands) 3, e.g. antibodies, exposed to a sample flow with analytes 4 (e.g. an antigen) through a flow channel 5. Monochromatic p-polarised light 6 from a light source 7 is coupled by a prism 8 to the glass/metal interface 9 where the light is totally reflected. The intensity of the reflected light beam 10 is detected by an optical detection unit 11.

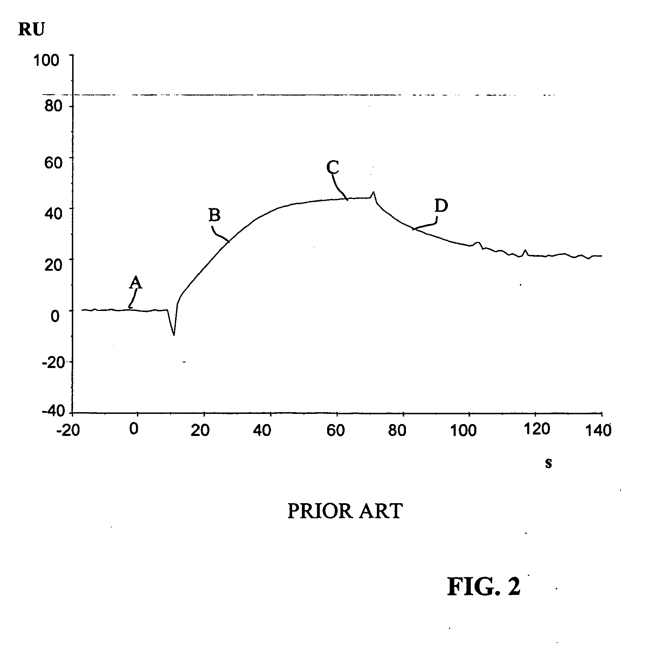

When molecules in the sample bind to the capturing molecules on the sensor chip surface, the concentration, and therefore the refractive index at the surface changes and an SPR response is detected. Plotting the response against time during the course of an interaction will provide a quantitative measure of the progress of the interaction. Such a plot is usually called a sensorgram. In the Biacore® system, the SPR response values are expressed in resonance units (RU). One RU represents a change of 0.00001° in the angle of minimum reflected light intensity, which for most proteins is roughly equivalent to a change in concentration of about 1 pg/mm² on the sensor surface. As sample containing an analyte contacts the sensor surface, the capturing molecule (ligand) bound to the sensor surface interacts with the analyte in a step referred to as "association." This step is indicated on the sensorgram by an increase in RU as the sample is initially brought into contact with the sensor surface. Conversely, "dissociation" normally occurs when sample flow is replaced by, for example, a buffer flow. This step is indicated on the sensorgram by a drop in RU over time as analyte dissociates from the surface-bound ligand. A representative sensorgram (binding curve) for a reversible interaction at the sensor chip surface is presented in Fig. 2, the sensing surface having an immobilized capturing molecule, for example an antibody, interacting with analyte in a sample. The vertical axis (y-axis) indicates the response (here in resonance units, RU) and the horizontal axis (x-axis) indicates the time (here in seconds, s). Initially, buffer is passed over the sensing surface giving the baseline response A in the sensorgram. During sample injection, an increase in signal is observed due to binding of the analyte. This part B of the binding curve is usually referred to as the "association phase". Eventually, a steady state condition is reached where the resonance signal plateaus at C. At the end of sample injection, the sample is replaced with a continuous flow of buffer and a decrease in signal reflects the dissociation, or release, of analyte from the surface. This part D of the binding curve is usually referred to as the "dissociation phase". The shape of the association/dissociation curve provides valuable information regarding the interaction kinetics, and the height of the resonance signal represents surface concentration (i.e., the response resulting from an interaction is related to the change in mass concentration on the surface).

Assume a reversible reaction (which is not mass transfer limited) between an analyte A and a surface-bound (immobilized) capturing molecule B (first order kinetics):

$$A + B \Leftrightarrow AB$$

The rate of change in surface concentration of A during analyte injection is

$$\frac{d\Gamma}{dt} = k_{ass}(\Gamma_{max} - \Gamma)C - k_{diss}\Gamma$$

where $\Gamma$ is the concentration of bound analyte, $\Gamma_{max}$ is the maximum binding capacity of the surface, $k_{ass}$ is the association rate constant, $k_{diss}$ is the dissociation rate constant, and C is the bulk analyte concentration. Rearrangement of the equation gives:

$$\frac{d\Gamma}{dt} = k_{ass}C\Gamma_{max} - (k_{ass}C + k_{diss})\Gamma$$

If all concentrations are measured in the same units, the equation may be rewritten as:

$$\frac{dR}{dt} = k_{ass}CR_{max} - (k_{ass}C + k_{diss})R$$

where R is the response in RU. In integrated form, the equation is:

$$R = \frac{k_{ass}CR_{max}}{k_{ass}C + k_{diss}}\left(1 - e^{-(k_{ass}C + k_{diss})t}\right)$$

The rate of dissociation can be expressed as:

$$\frac{dR}{dt} = -k_{diss}R$$

and in integrated form:

$$R = R_0 \cdot e^{-k_{diss}t}$$

Affinity is expressed by the association constant $K_A = k_{ass}/k_{diss}$ or the dissociation constant $K_D = k_{diss}/K_{ass}$.

For a more detailed description of the determination of molecular interaction kinetics, it may be referred to, for example, Karlsson, R. and Fält, A. (1997) Journal of Immunological Methods, 200, 121-133.

Now, if the sample is a "complex medium" which in addition to analyte contains one or more species that may also interact with the sensor surface, it is readily seen that the unspecific binding caused by the presence of such species may seriously disturb the measurements at the surface and give binding curves that are difficult to interpret, especially if the analyte of interest is a low molecular weight compound (usually referring to organic compounds having a molecular weight in the range of from about 100 to about 1000, typically from about 500 to about 800, sometimes also referred to as "small molecules").

According to the present invention, it has now been found that binding of even a low molecular weight molecule to an immobilized binder for the analyte on a sensor surface may be successfully measured when the analyte is present in a complex medium, if the sensor surface is contacted with complex sample medium free from analyte prior to contacting the surface with the analyte-containing sample medium. By proceeding in this way, non-specific binding to the sensor surface will primarily take place when the surface is contacted with the analyte-free complex medium. When the sample is then brought in contact with the sensor surface, the non-specific binding process has been at least substantially reduced, and the sample measurement will be disturbed to at least a much lesser extent, as will be demonstrated in the Example below.

Preferably, the sensor surface is again contacted with the analyte-free complex medium after the contact with the analyte-containing sample medium to permit dissociation of analyte from the immobilized analyte binder to take place in the same environment as the association to the binder. Thus, with reference to the above described Biacore® instrument, the liquid injection sequence of the invention would be <analyte-free sample medium>, <analyte-containing sample medium>, <analyte-free sample medium> in contrast to the prior art procedure of <buffer>, <analyte-containing sample medium>, <buffer>.

The complex medium upon which the sample is based may be selected from numerous such media containing one or more analytes of interest. Exemplary complex media include body fluids, such as cerebrospinal fluid, saliva, breast milk, urine, bile, blood serum or plasma, tears, homogenized biopsies, as well as other complex media such as cell culture media, cell lysates, crude plant extracts, extracted or dissolved food stuffs, liquid food stuffs, such as beverages (milk, fruit juices, beer etc).

Depending on the particular complex medium to be analyzed, the tested sample may be the original sample as taken or a dilution thereof with a suitable diluent. Generally, the complex medium content of the sample may range from about 1 to about 100 % (v/v), usually from about 10 to about 100 % (v/v), especially from about 30 to about 100 % (v/v), for example from about 30 to about 50 % (v/v).

When the complex sample medium, for example, is based on human blood plasma or serum, the analyte-containing medium may be based on plasma or serum taken from a patient after administration of a drug, and the analyte-free complex medium may then be based on plasma or serum taken from the same patient before the drug administration.

The method of the present invention may thus be used to determine kinetic and affinity constants for molecular interactions between an analyte in a complex medium and an immobilized binder for the analyte, including association constants, dissociation constants, association rate constants, and dissociation rate constants. The method of the present invention may, of course, also be used to determine the concentration of one or more analytes in a complex medium. Apart from contacting the binder-supporting surface with analyte-free complex medium prior to, and optionally after, contacting the surface with the analyte-containing complex medium, the determinations of kinetic and affinity constants as well as analyte concentration may be performed in *per se* known manner.

In a variant of the present invention, the method is used to compare the plasma binding propensity (such as e.g. affinity) of various drugs. More particularly, the binding of a drug to an immobilized receptor is determined, especially in the form of binding curves as described above, by contacting a surface having immobilized receptor with (i) drug dissolved in buffer, and (ii) drug dissolved in buffer with addition of blood plasma, and the binding levels, or preferably, the binding curves are then compared. If a drug binds to plasma, the resulting binding to the immobilized receptor in the presence of blood plasma would be substantially reduced as compared to the drug when present in buffer only. Measurements at a single drug concentration may often be sufficient.

While the description above has been made with some respect to the Biacore® systems, it is understood that the invention may be used in connection with numerous other techniques for detecting binding interactions at the solid support surface, including, e.g., those relying on a label, such as a radiolabel, a chromophore, a fluorophore, a marker for scattering light, an electrochemically active marker (e.g. field effect transistor based potentiometry), an electric field active marker (electro-stimulated emission), a magnetically active marker, a thermoactive marker, a chemiluminescent moiety or a transition metal, as well as so-called label free detection systems. Real time detection systems are, however, preferred, especially those based on chemical sensor or biosensor technology.

A biosensor is broadly defined as a device that uses a component for molecular recognition (for example a layer with immobilised antibodies) in either direct conjunction with a solid state physicochemical transducer, or with a mobile carrier bead/particle being in conjunction with the transducer. While such sensors are typically based on label-free techniques, detecting e.g. a change in mass, refractive index, or thickness for the immobilized layer, there are also sensors relying on some kind of labelling. Typical sensor detection techniques include, but are not limited to, mass detection methods, such as optical, thermo-optical and piezoelectric or acoustic wave (including e.g. surface acoustic wave (SAW) and quartz crystal microbalance (QCM)) methods, and electrochemical methods, such as potentiometric, conductometric, amperometric and capacitance/impedance methods. With regard to optical detection methods, representative methods include those that detect mass surface concentration, such as reflection-optical methods, including both external and internal reflection methods, which may be angle, wavelength, polarization, or phase resolved, for example evanescent wave ellipsometry and evanescent wave spectroscopy (EWS, or Internal Reflection Spectroscopy), both of which may include evanescent field enhancement via surface plasmon resonance (SPR), Brewster angle refractometry, critical angle refractometry, frustrated total reflection (FTR), scattered total internal reflection (STIR) which may include scatter enhancing labels, optical wave guide sensors; external reflection imaging, evanescent wave-based imaging such as critical angle resolved imaging, Brewster angle resolved imaging, SPR-angle resolved imaging, and the like. Further, photometric and imaging/microscopy methods, "per se" or combined with reflection methods, based on for example surface enhanced Raman spectroscopy (SERS), surface enhanced resonance Raman spectroscopy (SERRS), evanescent wave fluorescence (TIRF) and phosphorescence may be mentioned, as well as waveguide interferometers, waveguide leaky mode spectroscopy, reflective interference spectroscopy (RIfS), transmission interferometry, holographic spectroscopy, and atomic force microscopy (AFR).

Commercially available today are *inter alia* biosensor systems based on SPR. Exemplary such SPR-biosensors include the above-mentioned Biacore® instruments. A detailed discussion of the technical aspects of the Biacore® instruments and the phenomenon of SPR may be found in U.S. Patent No. 5,313,264. More detailed information on matrix coatings for biosensor sensing surfaces is given in, for example, U.S. Patents Nos. 5,242,828 and 5,436,161. In addition, a detailed discussion of the technical aspects of the biosensor chips used in connection with the Biacore® instruments may be found in U.S. Patent No. 5,492,840.

It may many times be convenient to carry out the method of the invention in a flow cell, e.g. of the type used in the above-mentioned Biacore® instruments. Other flow cells that may be used in the present invention are also well known to the skilled person and need not be described herein.

It is to be noted that the term "solid support" as used herein is to be interpreted broadly and is meant to comprise any solid (flexible or rigid) substrate onto which one or more binding agents can be immobilized and molecular interactions therewith be detected by the particular detection system chosen. The substrate may be biological, non-biological, organic, inorganic or a combination thereof, and may be in the form of particles, strands, precipitates, gels, sheets, tubings, spheres, containers, capillaries, pads, slices, films, plates, slides, etc, having any convenient shape, including disc, sphere, circle, etc.

The substrate surface may have any two-dimensional configuration and may include, for example steps, ridges, kinks, terraces and the like and may be the surface of a layer of material different from that of the rest of the substrate.

In the following Example, various aspects of the present invention are disclosed more specifically for purposes of illustration and not limitation.

EXAMPLE

This Example demonstrates the use of the method of the present invention to measure the interaction of iophenoxic acid ($\alpha$-ethyl-3-hydroxy-2,4,6-triiodohydro-cinnamic acid) in a saliva-containing buffer with immobilized human serum albumin (HSA). Iophenoxic acid is known to bind to HSA but not to IgG which therefore is used as a reference. The saliva components bind unspecifically to both HSA and IgG.

Instrumentation

A Biacore® 3000 instrument (Biacore AB, Uppsala, Sweden) was used. This instrument, which is based on surface plasmon resonance (SPR) detection at a gold surface, uses a micro-fluidic system for passing samples and running buffer through four individually detected flow cells, designated Fc1 to Fc4, one by one or in series. As sensor chip was used Series CM5 certified grade (Biacore AB, Uppsala, Sweden) which has a gold-coated surface with a covalently linked carboxymethyl-modified dextran polymer hydrogel. The output from the instrument is a "sensorgram" which is a plot of detector response (measured in "resonance units", RU) as a function of time. An increase of 1000 RU corresponds to an increase of mass on the sensor surface of approximately 1 ng/mm$^2$.

A prototype TRIINJECT command was used which injects two liquids, A and B, in the order <liquid A> <liquid B> <liquid A> (Andersson, Karl, et al. (1999) PROTEINS: Structure, Function, and Genetics 37, 494-498).

Sensor chip immobilization

Three of the flow channels of the Biacore® 3000 instrument, Fc1, Fc2 and Fc3, were used. Anti-myoglobin IgG (in-house reagent) and human serum albumin (HSA) (Sigma-Aldrich, Missouri, USA) were immobilized using Amine Coupling Kit (Biacore AB, Uppsala, Sweden) according to the manufacturer's instructions. Running buffer was 10 mM sodium-acetate buffer pH 5.0 (Biacore AB, Uppsala, Sweden). The sensor chip was immobilized as follows:

Fc1:    blank
Fc2:    about 8600 RU IgG (for reference use)
Fc3:    about 11000 RU Human Serum Albumin (HSA)
Fc4:    about 9000 RU Human Serum Albumin (HSA)

While sensorgrams were recorded for Fc1 to Fc4, only sensorgrams recorded for Fc2 and Fc3 were used for analysis.

Measurement of unspecific binding of saliva to IgG and HSA

The following two buffers were prepared:

HBS1%: HBS-EP (Biacore AB, Uppsala, Sweden) containing 1% DMSO (Riedel de Haën, Seelze, Germany).
HBSS1%: HBS-EP (Biacore AB) containing 1% DMSO (Riedel de Haën) and 30% saliva (donated by a healthy male).

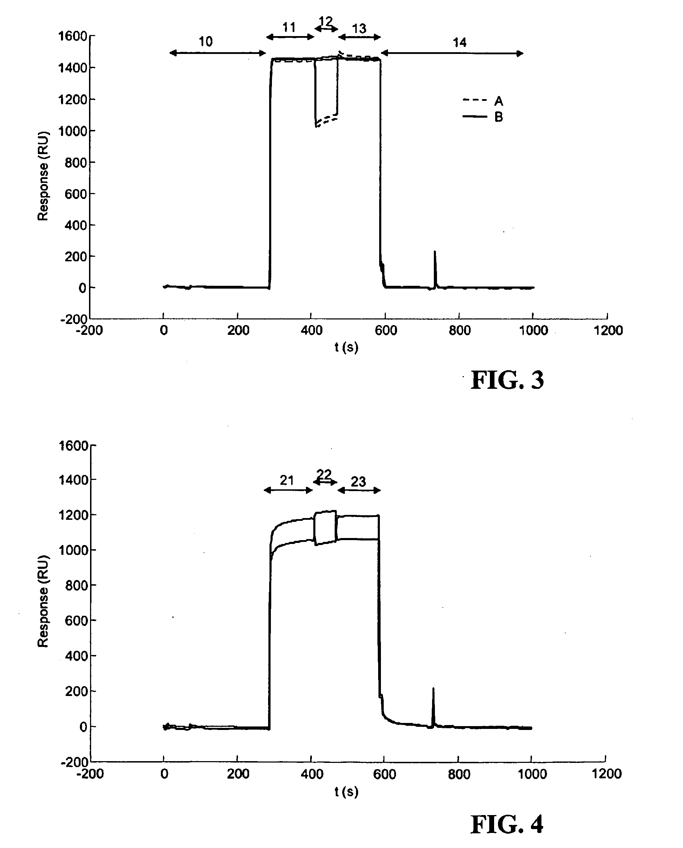

Using the trinject command of the Biacore® 3000 instrument, injections were made over flow cells 1 to 3 (Fc1, Fc2, Fc3) in series with liquid A being HBS1% and liquid B being either HBS1% or HBSS1%. That is, the injection sequences were <HBS1% > <HBS1% > <HBS1% >, and <HBS1% > <HBSS1% > <HBS1% >, respectively. Running buffer, used during preparatory and wash steps, was HBS-EP (Biacore AB, Uppsala, Sweden). The sensorgrams obtained for the HSA-surface (Fc3) are shown as overlay plots in Fig. 3. The dashed line, designated A in the figure, shows the sensorgram when liquid B in the triinject is HBSS1%, and the solid line, designated B in the figure, shows the sensorgram when liquid B in the triinject is HBS1%.

During segment 10 in Fig. 3, the instrument prepares for injection with running buffer. Segments 11 and 13 are <liquid A> in the triinject, in this case HBS1%. Segment 12 is the actual sample injection, i.e. <liquid B> in the triinject. 12A corresponds to HBSS1% and 12B corresponds to HBS1%. Segment 14 is wash with running buffer. The sudden shift of response level (RU) in the sensorgrams in each segment transition reflects the different bulk refractive index of the respective liquids, and the slower changes of the response level during each segment corresponds to a specific or unspecific interaction. When comparing segments 12A and 12B, segment 12A has a significantly larger slope than that of 12B, indicating that the saliva components in 12A bind to the surface.

In a similar experiment, a sample injection of HBSS1% was "embedded" in HBSS 1 %, i.e. <liquid A> and <liquid B> were both HBSS1%. The sensorgrams from two such injections are shown as overlay plots in Fig. 4 (signal from flow cell 3 as in Fig. 3), segments 21 to 23 corresponding to segments 11 to 13 in Fig. 3. As seen in Fig. 4, there is a large drift early in the segment 21 due to unspecific components binding rapidly. At the time of transition from segment 21 to 22, the drift has decreased. In particular, the drift during segment 22 is lower than that during segment 12, demonstrating that if the surface is already preincubated with the unspecific components when the sample is injected, the signal is not influenced by unspecific binding to the same extent.

<u>Measurement of interaction of iophenoxic acid in 30% saliva with HSA</u>

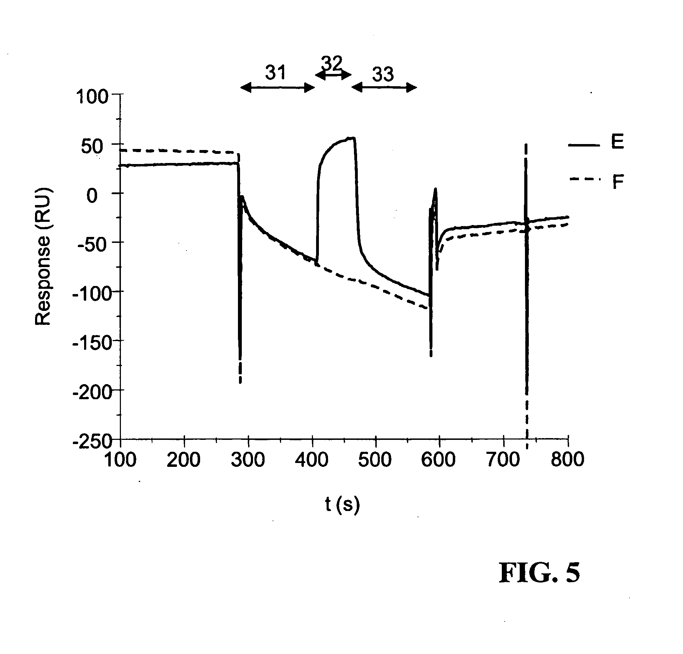

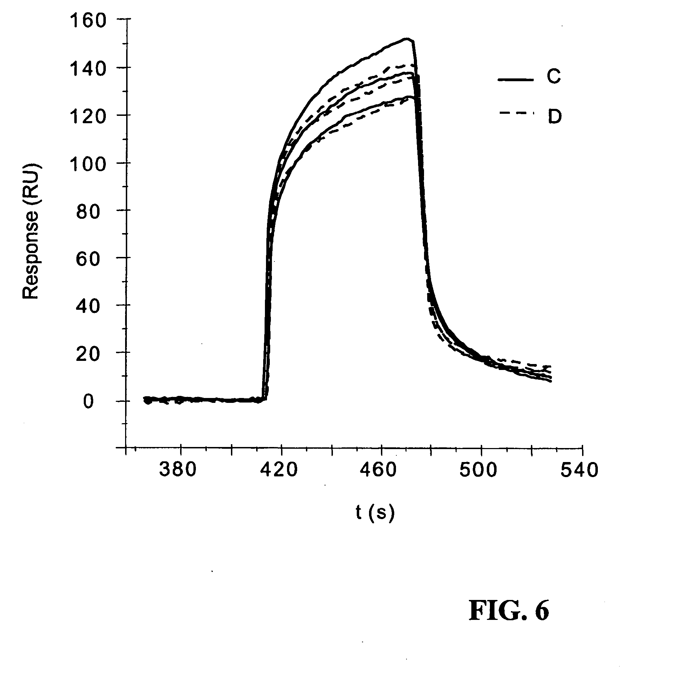

50 μM iophenoxic acid (Sigma-Aldrich, Missouri, USA) in HBSS 1% was used as <liquid B>, and HBSS1% as <liquid A>, and triinject injects were performed over Fc1, Fc2 and Fc3 in series. Detection was performed at Fc3 (HSA-surface) and Fc2 (IgG-surface). The sensorgrams obtained for Fc3 were "corrected" by subtracting the sensorgram for the reference, Fc2. The reference subtracted sensorgrams (Fc3 - Fc2) obtained are shown as overlay plots in Fig. 5. The solid line, designated E in the figure, shows the sensorgram when liquid B in the triinject is 50 μM iophenoxic acid in HBSS1%, and the dashed line, designated F in the figure, shows the sensorgram when liquid B in the triinject is HBSS 1%.
In Fig. 5, segments 31, 32F and 33 are HBSS1%, and segment 32E is 50 μM iophenoxic acid in HBSS1%. The slope during segments 31-33 is due to saliva components binding unspecifically to IgG (Fc2) at a lower rate than to HSA (Fc3). The influence of unspecific binding is, however, stable when the iophenoxic acid is injected in segment 32. As seen from segments 32E and 32F, the binding of iophenoxic acid can readily be quantified.
To correct for the difference of unspecific binding between IgG and HSA, the sensorgram from the buffer injection (F in Fig. 5) may be subtracted from the sensorgram from the iophenoxic acid injection (E in Fig. 5). The binding of iophenoxic acid to HSA was characterized three times in HBSS1% and three times in HBS1%, and the resulting sensorgrams corrected in this way are shown in Fig. 6. The sensorgrams for the saliva-containing buffer are shown in dashed lines (D), while the sensorgrams for the pure buffer are shown in solid lines (C). As seen in Fig. 6, sensorgrams D (saliva-containing buffer) agree with the sensorgrams C (pure buffer), indicating that this method is favourable when characterizing molecular interactions in complex matrices.
Thus, by preincubating the HSA and IgG surfaces with unspecifically binding saliva components prior to contacting the surfaces with the sample, unspecific binding from the sample medium which would disturb the measurement of the iophenoxic acid interaction is minimized. The method of the invention is therefore suitable for minimizing the influence of possible unspecific binding in mesurements of how especially small molecules bind to a ligand in a complex sample medium.
It is to be understood that the invention is not limited to the particular embodiments of the invention described above, but the scope of the invention will be established by the appended claims.

## Claims

1. A method for determining interaction of an analyte with a binding agent immobilized to a solid support surface by contacting the surface with a fluid sample containing the analyte, and detecting binding events at the solid support surface, wherein the sample is based on a complex medium containing at least one species other than analyte that may interact with the solid support surface, **characterized in that** the method comprises contacting the solid support surface with sample medium free from analyte prior to contacting the surface with the analyte-containing sample.

2. The method according to claim 1, **characterized in that** association of analyte to the immobilized binding agent is determined.

3. The method according to claim 1 or 2, **characterized in that** the solid support surface, after having been contacted with the analyte-containing sample, is contacted again with sample medium free from analyte and dissociation of analyte from the surface is determined.

4. The method according to any one of claims 1 to 3, **characterized in that** at least one of the association constant, dissociation constant, association rate constant and dissociation rate constant for the interaction is determined.

5. The method according to any one of claims 1 to 4, **characterized in that** the concentration of analyte in the sample is determined.

6. The method according to any one of claims 1 to 5, **characterized in that** the sample comprises a complex medium diluted with a diluent.

7. The method according to any one of claims 1 to 6, **characterized in that** the complex medium is a body fluid.

8. The method according to claim 7, **characterized in that** the body fluid is selected from cerebrospinal fluid, saliva, breast milk, urine, bile, blood serum, blood plasma, tears, and homogenized biopsies, preferably from blood plasma and blood serum, particularly from human blood plasma and human blood serum.

9. The method according to any one of claims 1 to 6, **characterized in that** the complex medium is selected from cell culture media, cell lysates, crude plant extracts, liquid food stuff, extracted food stuff, and dissolved food stuff.

**10.** The method according to any one of claims 1 to 9, **characterized in that** the complex medium content in the sample is from 1 to 100%, preferably from 10 to 100%, more preferably from 30 to 100%, particularly from 30 to 50%.

**11.** The method according to any one of claims 1 to 10, **characterized in that** the analyte is a low molecular weight compound.

**12.** The method according to any one of claims 1 to 11, **characterized in that** the sample is based on blood plasma or serum taken from a patient after a drug has been administered to the patient, and wherein the sample medium free from analyte is based on blood plasma or serum taken from the patient before the administration of the drug.

**13.** The method according to any one of claims 1 to 12, **characterized in that** the solid support surface comprises a sensor surface, and binding to the surface causes a measurable change of a characteristic of the sensor surface.

**14.** The method according to any one of claims 1 to 13, **characterized in that** the binding events at the solid support surface are detected in real time.

**15.** The method according to claim 14, **characterized in that** the binding events at the solid support surface are represented by a binding curve for detected binding level versus time.

**16.** The method according to any one of claims 13 to 15, **characterized in that** the sensor surface is part of a biosensor.

**17.** The method according to claim 16, **characterized in that** the biosensor is based on evanescent wave sensing, preferably surface plasmon resonance (SPR).

**18.** The method according to any one of claims 1 to 17, **characterized in that** the method is performed in a flow cell.

**19.** A method for determining the plasma binding propensity of a drug, which method comprises determining the binding of a drug (i) in a complex medium comprising blood plasma and (ii) in a non-complex medium, to an immobilized binder for the drug, and comparing the binding of drug in the blood plasma-comprising medium with the binding of drug in the non-complex medium, **characterized in that** the determination of the binding of drug in the blood plasma-comprising medium is performed according to the method of any one of claims 1 to 18.

**Patentansprüche**

**1.** Verfahren zur Bestimmung der Wechselwirkung eines Analyten mit einem an einer Festträgeroberfläche immobilisierten Bindungsmittel durch In-Kontakt-Bringen der Oberfläche mit einer fluiden Probe, die den Analyten enthält, und Nachweisen von Bindungsereignissen an der Festträgeroberfläche, wobei die Probe auf einem Komplexmedium basiert, das zumindest eine Spezies außer dem Analyten enthält, die mit der Festträgeroberfläche in Wechselwirkung stehen kann, **dadurch gekennzeichnet, dass** das Verfahren das In-Kontakt-Bringen der Festträgeroberfläche mit analytfreiem Probenmedium vor dem In-Kontakt-Bringen der Oberfläche mit der analythaltigen Probe umfasst.

**2.** Verfahren nach Anspruch 1, **dadurch gekennzeichnet, dass** die Anbindung des Analyten an das immobilisierte Bindungsmittel bestimmt wird.

**3.** Verfahren nach Anspruch 1 oder 2, **dadurch gekennzeichnet, dass** die Festträgeroberfläche, nachdem sie mit der analythaltigen Probe in Kontakt gebracht worden ist, erneut mit analytfreiem Probenmedium in Kontakt gebracht wird und die Dissoziation des Analyten von der Oberfläche bestimmt wird.

**4.** Verfahren nach einem der Ansprüche 1 bis 3, **dadurch gekennzeichnet, dass** von der Assoziationskonstante, der Dissoziationskonstante, der Assoziationsratenkonstante und der Dissoziationsratenkonstante für die Wechselwirkung zumindest eine bestimmt wird.

**5.** Verfahren nach einem der Ansprüche 1 bis 4, **dadurch gekennzeichnet, dass** die Analytkonzentration in der Probe bestimmt wird.

**6.** Verfahren nach einem der Ansprüche 1 bis 5, **dadurch gekennzeichnet, dass** die Probe ein mit einem Verdünnungsmittel verdünntes Komplexmedium umfasst.

**7.** Verfahren nach einem der Ansprüche 1 bis 6, **dadurch gekennzeichnet, dass** das Komplexmedium eine Körperflüssigkeit ist.

**8.** Verfahren nach Anspruch 7, **dadurch gekennzeichnet, dass** die Körperflüssigkeit gewählt wird aus Gehirn-Rückenmark-Flüssigkeit, Speichel, Muttermilch, Urin, Galle, Blutserum, Blutplasma, Tränen und homogenisierten Biopsaten, bevorzugt aus Blutplasma und Blutserum, insbesondere aus menschlichem Blutplasma und menschlichem Blutserum.

**9.** Verfahren nach einem der Ansprüche 1 bis 6, **dadurch gekennzeichnet, dass** das Komplexmedium gewählt wird aus Zellkulturmedien, Zelllysaten, Pflanzenrohextrakten, flüssigen Lebensmitteln, extrahierten Lebensmitteln und gelösten Lebensmitteln.

10. Verfahren nach einem der Ansprüche 1 bis 9, **dadurch gekennzeichnet, dass** der Komplexmediumgehalt in der Probe 1 bis 100%, bevorzugt 10 bis 100%, besonders bevorzugt 30 bis 100% und ganz besonders bevorzugt 30 bis 50% beträgt.

11. Verfahren nach einem der Ansprüche 1 bis 10, **dadurch gekennzeichnet, dass** der Analyt eine niedermolekulare Verbindung ist.

12. Verfahren nach einem der Ansprüche 1 bis 11, **dadurch gekennzeichnet, dass** die Probe auf Blutplasma oder -serum basiert, das einem Patienten entnommen wird, nachdem ein Wirkstoff dem Patienten verabreicht worden ist, und wobei das analytfreie Probenmedium auf Blutplasma oder -serum basiert, das dem Patienten vor der Verabreichung des Wirkstoffs entnommen wird.

13. Verfahren nach einem der Ansprüche 1 bis 12, **dadurch gekennzeichnet, dass** die Festträgeroberfläche eine Sensoroberfläche umfasst, und eine Bindung an die Oberfläche eine messbare Veränderung einer Charakteristik der Sensoroberfläche bewirkt.

14. Verfahren nach einem der Ansprüche 1 bis 13, **dadurch gekennzeichnet, dass** die Bindungsereignisse an der Festträgeroberfläche in Echtzeit nachgewiesen werden.

15. Verfahren nach Anspruch 14, **dadurch gekennzeichnet, dass** die Bindungsereignisse an der Festträgeroberfläche dargestellt werden durch eine Bindungskurve für nachgewiesenen Bindungsgrad versus Zeit.

16. Verfahren nach einem der Ansprüche 13 bis 15, **dadurch gekennzeichnet, dass** die Sensoroberfläche Teil eines Biosensors ist.

17. Verfahren nach Anspruch 16, **dadurch gekennzeichnet, dass** der Biosensor auf Wahrnehmung evaneszenter Wellen, vorzugsweise Oberflächenplasmonenresonanz (SPR: Surface Plasmon Resonance), beruht.

18. Verfahren nach einem der Ansprüche 1 bis 17, **dadurch gekennzeichnet, dass** das Verfahren in einer Flusszelle durchgeführt wird.

19. Verfahren zur Bestimmung der Plasmabindungstendenz eines Wirkstoffs, das Verfahren umfassend das Bestimmen der Bindung eines Wirkstoffs (i) in einem Blutplasma umfassenden Komplexmedium und (ii) in einem Nicht-Komplexmedium, an einen immobilisierten Binder für den Wirkstoff, und das Vergleichen der Bindung des Wirkstoffs im Blutplasma umfassenden Medium mit der Bindung des Wirkstoffs im Nicht-Komplexmedium, **dadurch gekennzeichnet, dass** die Bestimmung der Bindung des Wirkstoffs im Blutplasma umfassenden Medium durchgeführt wird gemäß dem Verfahren nach einem der Ansprüche 1 bis 18.

## Revendications

1. Procédé de détermination de l'interaction d'un analyte avec un agent de liaison immobilisé sur une surface de support solide par la mise en contact de la surface avec un échantillon fluide contenant l'analyte, et la détection d'événements de liaison au niveau de la surface de support solide, dans lequel l'échantillon est basé sur un milieu complexe contenant au moins une espèce autre que l'analyte qui peut interagir avec la surface de support solide, **caractérisé en ce que** le procédé comprend la mise en contact de la surface de support solide avec un milieu d'échantillon exempt d'analyte avant la mise en contact de la surface avec l'échantillon contenant l'analyte.

2. Procédé selon la revendication 1, **caractérisé en ce que** l'association d'un analyte à l'agent de liaison immobilisé est déterminée.

3. Procédé selon la revendication 1 ou 2, **caractérisé en ce que** la surface de support solide, après avoir été mise en contact avec l'échantillon contenant l'analyte, est de nouveau mise en contact avec le milieu d'échantillon exempt d'analyte et la dissociation d'analyte de la surface est déterminée.

4. Procédé selon l'une quelconque des revendications 1 à 3, **caractérisé en ce que** au moins l'une de la constante d'association, la constante de dissociation, la constante de vitesse d'association et la constante de vitesse de dissociation de l'interaction est déterminée.

5. Procédé selon l'une quelconque des revendications 1 à 4, **caractérisé en ce que** la concentration d'analyte dans l'échantillon est déterminée.

6. Procédé selon l'une quelconque des revendications 1 à 5, **caractérisé en ce que** l'échantillon comprend un milieu complexe dilué avec un diluant.

7. Procédé selon l'une quelconque des revendications 1 à 6, **caractérisé en ce que** le milieu complexe est un fluide corporel.

**8.** Procédé selon la revendication 7, **caractérisé en ce que** le fluide corporel est sélectionné parmi le fluide cérébro-spinal, la salive, le lait maternel, l'urine, la bile, le sérum sanguin, le plasma sanguin, les larmes, et les biopsies homogénéisées, de préférence, parmi le plasma sanguin et le sérum de sanguin, plus particulièrement parmi le plasma de sang humain et le sérum de sang humain.

**9.** Procédé selon l'une quelconque des revendications 1 à 6, **caractérisé en ce que** le milieu complexe est sélectionné parmi des milieux de culture cellulaire, des lysats cellulaires, des extraits de plantes naturelles, des denrées alimentaires liquides, des denrées alimentaires extraites, et des denrées alimentaires dissoutes.

**10.** Procédé selon l'une quelconque des revendications 1 à 9, **caractérisé en ce que** la teneur en milieu complexe dans l'échantillon est comprise entre 1 et 100 %, de préférence, entre 10 et 100 %, plus préférablement, entre 30 et 100 %, plus particulièrement, entre 30 et 50 %.

**11.** Procédé selon l'une quelconque des revendications 1 à 10, **caractérisé en ce que** l'analyte est un composé de faible poids moléculaire.

**12.** Procédé selon l'une quelconque des revendications 1 à 11, **caractérisé en ce que** l'échantillon est basé sur du plasma ou sérum sanguin prélevé sur un patient après qu'un médicament a été administré au patient, et dans lequel le milieu d'échantillon exempt d'analyte est basé sur du plasma ou sérum sanguin prélevé sur le patient avant l'administration du médicament.

**13.** Procédé selon l'une quelconque des revendications 1 à 12, **caractérisé en ce que** la surface de support solide comprend une surface de capteur, et la liaison à la surface provoque une variation mesurable d'une caractéristique de la surface de capteur.

**14.** Procédé selon l'une quelconque des revendications 1 à 13, **caractérisé en ce que** les événements de liaison au niveau de la surface de support solide sont détectés en temps réel.

**15.** Procédé selon la revendication 14, **caractérisé en ce que** les événements de liaison au niveau de la surface de support solide sont représentés par une courbe de liaison du niveau de liaison détecté en fonction du temps.

**16.** Procédé selon l'une quelconque des revendications 13 à 15, **caractérisé en ce que** la surface de capteur est une partie d'un biocapteur.

**17.** Procédé selon la revendication 16, **caractérisé en ce que** le biocapteur est basé sur la détection d'onde évanescente, de préférence, la résonance de plasmons de surface (SPR).

**18.** Procédé selon l'une quelconque des revendications 1 à 17, **caractérisé en ce que** le procédé est mis en oeuvre dans une cellule d'écoulement.

**19.** Procédé de détermination de la capacité à se lier au plasma d'un médicament, lequel procédé comporte la détermination de la liaison d'un médicament (i) dans un milieu complexe comprenant du plasma sanguin et (ii) dans un milieu non complexe, à un lieur immobilisé pour le médicament, et la comparaison de la liaison de médicament dans le milieu comprenant du plasma sanguin avec la liaison de médicament dans le milieu non complexe, **caractérisé en ce que** la détermination de la liaison de médicament dans le milieu comprenant du plasma sanguin est réalisée suivant le procédé selon l'une quelconque des revendications 1 à 18.

PRIOR ART

**FIG. 1**

PRIOR ART

FIG. 2

**FIG. 3**

**FIG. 4**

**FIG. 5**

**FIG. 6**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5313264 A
- US 5242828 A
- US 5436161 A
- US 5492840 A

### Non-patent literature cited in the description

- **Karlsson, R. ; Fält, A.** *Journal of Immunological Methods,* 1997, vol. 200, 121-133
- **Andersson, Karl et al.** *PROTEINS: Structure, Function, and Genetics,* 1999, vol. 37, 494-498